# EUROPEAN PATENT APPLICATION

(11) **EP 3 862 630 A1**
(43) Date of publication of application: **11.08.2021**
(21) Application number: 20155337.7
(22) Date of filing: 04.02.2020
(51) Int. Cl.: F23D 3/24, F23D 3/40, A61L 9/03

(54) **AROMA DIFFUSER AND FRAGRANCE CAPSULE**

(71) Applicant: Dongguan Yih Teh Electric Products Co., Ltd, DongGuan City, GuangDong (CN)
(72) Inventor: HSIAO, Ming Jen, 35147 Toufen, Miaoli County (TW)
(74) Representative: Viering, Jentschura & Partner mbB Patent- und Rechtsanwälte

(57) **Abstract**

An aroma diffuser includes a housing, a burner, a thermal conducting device and a hollow annular fragrance capsule. The burner and the thermal conducting device are combined in the housing. The burner provides a complete combustion heat source to avoid contaminating the air. The thermal radiation of the thermal conducting device can be continuously and effectively transmitted to the donut-shaped fragrance capsule and the fragrance substance to quickly produce a stable aroma. The fragrance capsule is provided with a breathable film that prevents the fragrance substance from flowing to the outside of the fragrance capsule to contaminate the burner or the surroundings. The breathable film is formed of a fixing film and a microporous film. The fixing film combined with the microporous film is not deformed or ugly when heated, and the aroma can be diffused outward from the breathable film. The fragrance substance can be quickly replaced with another fragrance capsule after use.

## Description

### BACKGROUND OF THE INVENTION

### 1. Field of the Invention:

The present invention relates to scent releasing and heating devices and more particularly, to an aroma diffuser using fragrance capsule.

### 2. Description of the Related Art:

Conventional aroma diffusers include, for example, the use of candles to heat fragrance substances. The smoke generated by candle wick cotton causes problems such as TVOC or PM2.5 air pollution. Since the wick cotton will become short when burned, it is easy to extinguish after burning. When the fuel cup fuel stock becomes smaller, the flame will extinguish. In addition to the problem of burning carbon deposits, the flame of the burning of cotton wick is unstable. The aroma diffusing effect by the use of candles to heat fragrance substances is also unstable.

In prior art aroma diffuser designs invented by the present inventor, such as US Pat. No. 8,668,885, US Pat. No. 9,550,358, US Pat No. 9,498, 533, EP Pat. No. 2679249, a container is used to transmit a heat source to a fragrance capsule that is provided with a breathable film. The breathable film is a plastic film with air holes. However, after a period of use, the breathable film will deform and become ugly on the surface, or even affect the dissipation of the fragrance molecules of the fragrance substance through the air holes of the breathable film to the outside air. Furthermore, because the fragrance substance in the fragrance capsule is a solid wax block, the middle part of the solid wax block is difficult to be uniformly heated to effectively produce aroma.

### SUMMARY OF THE INVENTION

The present invention has been accomplished under the circumstances in view. It is thereof the main object of the present invention to provide an aroma diffuser using fragrance capsule, which provides a combustion heat source that can avoid polluting the air, and at the same time, the combustion heat source can be continuously and effectively transmitted to the fragrance substance of the fragrance capsule to generate a stable aroma.

To achieve this and other objects of the present invention, an aroma diffuser using fragrance capsule comprises a housing, a burner and a thermal conducting device. The housing comprising an opening, an accommodation space, a plurality of air holes and a locating portion. The opening is formed in a top side of the housing. The accommodation space is defined in the housing in communication with the opening. The air holes are formed on the periphery of the housing below the elevation of the opening. The locating portion is formed inside the housing below the air holes. The burner comprises a disc-like burner base and a wick. The disc-like burner base comprises a wick hole and a support tube. The support tube is upwardly extended from the disc-like burner base around the wick hole. The wick is inserted into the support tube and the wick hole. The burner is detachably mounted on the locating portion in the accommodation space of the housing. The burner defines with an inner bottom wall of the housing a fuel trough therebetween for the filling of a liquid fuel. The wick has one end thereof inserted into the fuel trough and an opposite end thereof extended out of the support tube and suspending in the accommodation space between the air holes. The thermal conducting device is detachably mounted on the burner in the accommodation space of the housing. The liquid fuel in the embodiment is preferable alcohol of gasoline that can be completely burned without polluting the air. The thermal conducting device (5) is used to heat the fragrance substance of the fragrance capsule for dissipating aroma into the air.

In some embodiments of the present invention, the thermal conducting device comprises a first annular container. The first annular container comprises a first opening, a first center tube and an inner flange. The first opening is formed in a top side of the first annular container. The inner flange horizontally projects from an inside wall of the first annular container. The first center tube upwardly projects from an inner bottom wall of the first annular container and is provided with an outer flange around the periphery thereof. The first center tube and the first annular container define therebetween a first annular groove.

In some embodiments of the present invention, the aroma diffuser further comprises a fragrance capsule. The fragrance capsule comprises a second annular container, a fragrance substance and a breathable film. The second annular container comprises a second opening and a second center tube. The second opening is formed in the top side of the second annular container. The second center tube has a top end thereof terminating in a nozzle. The second center tube upwardly projects from an inner bottom all of the second annular container. The second center tube and the second annular container define therebetween a second annular groove. The fragrance substance is mounted in the second annular groove. The breathable film is bonded to the second opening of the second annular container to cover the fragrance substance. The breathable film is breathable and waterproof and bonded to the second opening of the second annular container to cover the fragrance substance. The fragrance capsule is shaped like a donut for placing in the first annular container of the thermal conducting device conveniently. The second annular container is placed in the first annular container of the thermal conducting device for heating. The hollow part of the donut-like fragrance substance can be evenly heated to generate aroma, improving the problem of uneven heating of the conventional fragrance block. The thermal conducting device and the fragrance capsule can be integrally taken out of the housing. In this way, the liquid fuel can be supplemented to the disc-like burner base and flow through the first gap, then penetrate into the second gap and drip into the fuel trough via the drip holes.

During the use of the aroma diffuser, the burner can completely burn the liquid fuel, and the metal mesh wick will not be carbonized to cause pollution. The fixing film combined with the microporous film is not deformed or ugly when heated, and the aroma can be diffused outward from the breathable film. The fragrance substance gas molecules maintain good breathability with this breathable film to stably dissipate to outside space. The fragrance capsule is safe to use. After the evaporation of the fragrance substance, the fragrance capsule can be discarded and replaced view a new one.

### BRIEF DESCRIPTION OF THE DRAWINGS

FIG. 1 is an oblique top elevational view of an aroma diffuser using fragrance capsule in accordance with the present invention.
FIG. 2 is an exploded view of the aroma diffuser using fragrance capsule in accordance with the present invention.
FIG. 3 corresponds to FIG. 2 when viewed from another angle.
FIG. 4 illustrates the outer appearance of the aroma diffuser using fragrance capsule in accordance with the present invention.
FIG. 5 is a sectional view taken along line A-A of FIG. 4.
FIG. 6 is a sectional view, in an enlarged scale, of the wick.
FIG. 7 is a sectional view of a part of the present invention, illustrating the elastic cover fastened to the housing/

### DETAILED DESCRIPTION OF THE PREFERRED EMBODIMENT

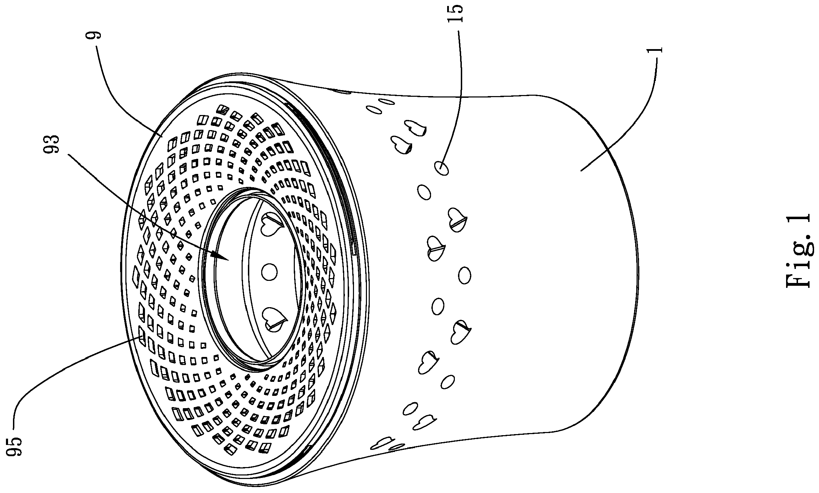

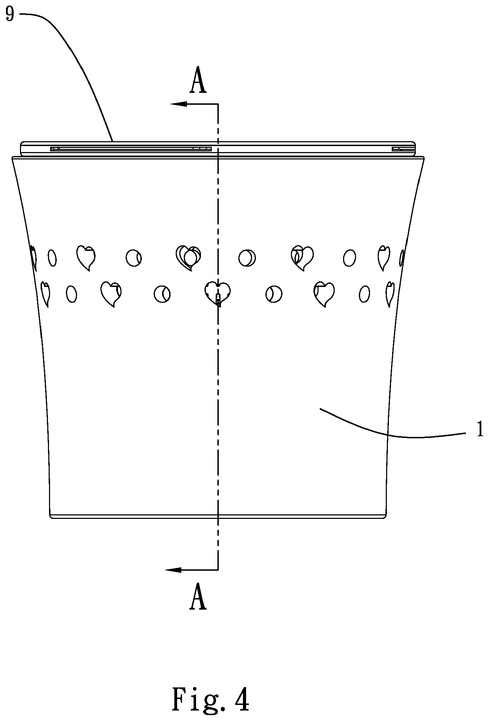

Referring to FIGS. 1-5, an aroma diffuser using fragrance capsule in accordance with the present invention is shown. The aroma diffuser using fragrance capsule comprises a housing (1), a burner (3) and a thermal conducting device (5).

The housing (1) comprises an opening (11), an accommodation space (13), air holes (15), and a locating portion (17). The opening (11) is formed on a top side of the housing (1). The space inside the housing (1) in communication with the opening (11) is defined as an accommodation space (13). The air holes (15) is formed on the periphery of the housing (1) below the opening (11). The locating portion (17) is formed inside the housing (1) under the air holes (15).

The burner (3) comprises a disc-like burner base (31) and a wick (33). The disc-like burner base (31) comprises a wick hole (311), and a support tube (313) upwardly extended from the periphery of the wick hole (311). The wick (33) is inserted into the support tube (313) and the wick hole (311). The burner (3) is detachably joined to the locating portion (17) in the accommodation space (13) of the housing (1). A fuel trough (19) is defined between the burner (3) and an inner bottom side of the housing (1). The wick (33) has one end thereof inserted into the fuel trough (19), and an opposite end thereof suspending between the air holes (15).

The thermal conducting device (5) is detachably mounted above the burner (3) in the accommodation space (13). The fuel trough (19) is used to fill a liquid fuel (191). In this embodiment, the liquid fuel is preferably alcohol, which can be completely burned without polluting the air.

In some embodiments, the burner (3) can also use various refined liquid fuels (191) such as kerosene, gasoline, vegetable oil, animal oil and the like.

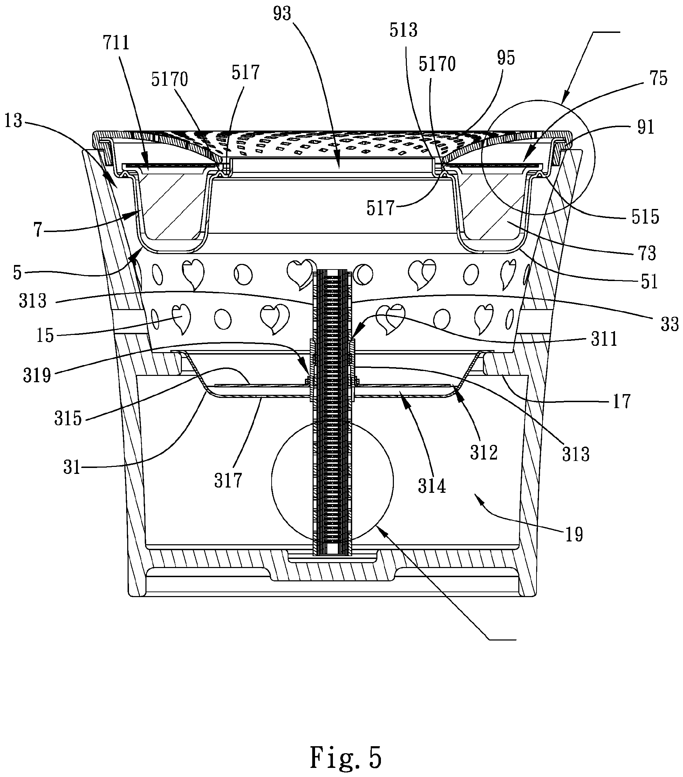

Referring to FIG. 5, the aroma diffuser of the present invention further comprises a fragrance capsule (7). The fragrance capsule (7) holds therein a fragrance substance (73). The fragrance capsule (7) is detachably placed in the thermal conducting device (5). The burner (3) heats the thermal conducting device (5). The thermal conducting device (5) conveys the heat source to the fragrance capsule (7) to heat the fragrance substance (73), causing the fragrance (73) to release fragrance.

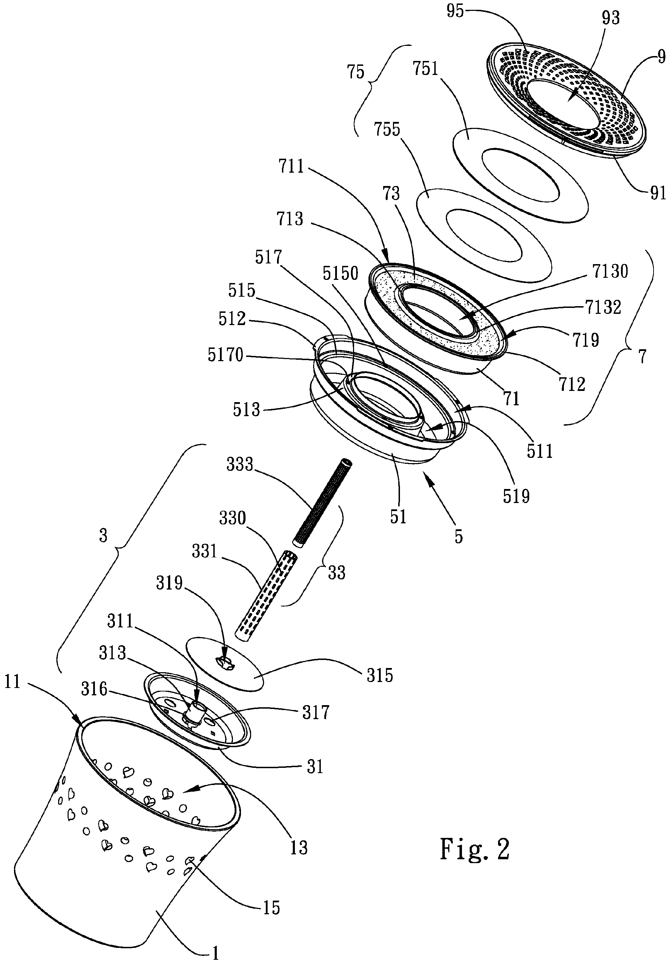

Referring to FIGS. 2, 3 and 5, in one embodiment of the present invention, the disc-like burner base (31) further comprises a cover sheet (315) and drip holes (317). The drip holes (317) are located at a bottom side of the disc-like burner base (31). The cover sheet (315) has a center hole (319). The support tube (313) has a flange (316) extended around the periphery thereof above the wick hole (311). The center hole (319) of the cover sheet (315) is attached onto the support tube (313) so that the cover sheet (315) can be supported on the flange (316). A first gap (312) is left between the periphery of the cover sheet (315) and an inner bottom wall of the disc-like burner base (31). A second gap (314) is left between the cover sheet (315) and the drip holes (317). Thus, the liquid fuel filled in the disc-like burner base (31) flows through the first gap (312), and then penetrates into the second gap (314) and drips into the fuel trough (19) via the drip holes (317).

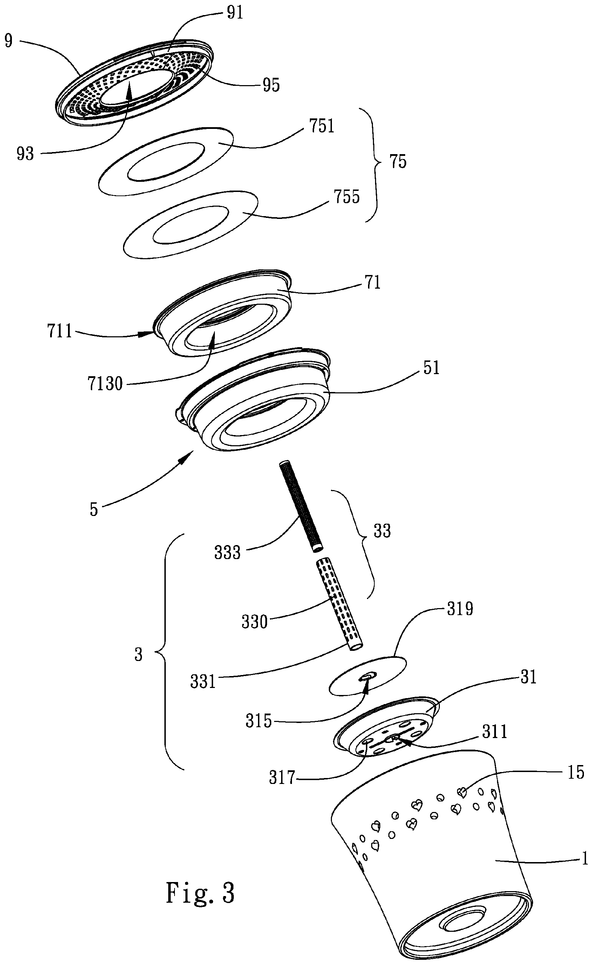

Referring to FIGS. 2 and 3, in one embodiment of the present invention, the wick (33) comprises a tube sleeve (331) and a metal mesh rod (333). The tube sleeve (331) has a plurality of holes (330) formed in the tube wall thereof. The metal mesh rod (333) is mounted in the tube sleeve (331). The metal mesh rod (333) contains a plurality of tiny meshes forming connected pores, which use capillary action to adsorb the liquid fuel (191) such as alcohol in the fuel trough (19) and deliver it to the area between the top side of the wick (33) and the air holes (15) above the disc-like burner base (31) for burning to heat the thermal conducting device (5). The wick (33) using the metal mesh rod (333) does not cause carbon pollution.

In some embodiments of the present invention, the metal mesh rod (333) is a wick structure with a metal mesh wound around a central axis, or a wick structure formed by folding or stacking a metal mesh into shape.

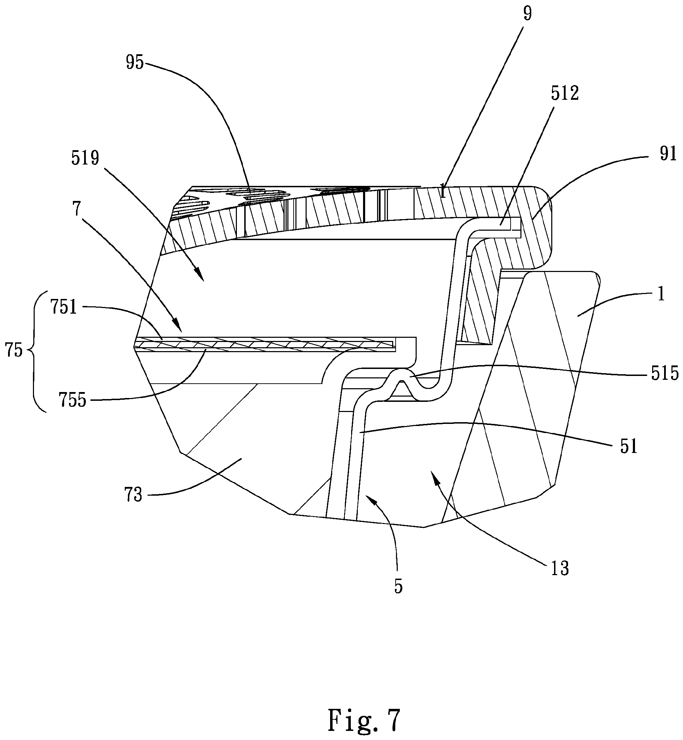

Referring to FIGS. 2, 3, 5 and 7, in one embodiment of the present invention, the thermal conducting device (5) comprises a first annular container (51). The first annular container (51) comprises a first opening (511), a first center tube (513) and an inner flange (515). The first opening (511) is formed on a top side of the first annular container (51). The inner flange (515) projects horizontally from an inner wall of the first annular container (51). The first center tube (513) projects upward from an annular hollow structure at a bottom side of the first annular container (51). The first center tube (513) is provided with an outer flange (517). The space between the first center tube (513) and the first annular container (51) defines a first annular groove (519).

Referring to FIGS. 2, 3, 5 and 7, in one embodiment of the present invention, the aroma diffuser further comprises a fragrance capsule (7) detachably placed in the thermal conducting device (5). The fragrance capsule (7) comprises a second annular container (71), a fragrance substance (73) and a breathable film (75). The second annular container (71) comprises a second opening (711) and a second center tube (713). The second opening (711) is formed on a top side of the second annular container (71). The second center tube (713) has a top end thereof terminating in a nozzle (7130). The second center tube (713) projects upward from an annular hollow structure at a bottom side of the second annular container (71). The space between the second center tube (713) and the second annular container (71) defines a second annular groove (719). The fragrance substance (73) is placed in the second annular groove (719). The breathable film (75) is fastened to the second opening (711) of the second annular container (71) and covered over the top side of the fragrance substance (73). The breathable film (75) has breathable and waterproof functions. The second annular container (71) of the fragrance capsule (7) is shaped like a donut and placed in the first annular groove (519) of the first annular container (51) of the thermal conducting device (5). The donut-shaped fragrance substance (73) and the annular hollow structure of the second annular container (71) can be evenly heated to generate fragrance, eliminating the problem of uneven heating of the conventional fragrance block. The thermal conducting device (5) and the fragrance capsule (7) can be integrally taken away from the housing (1). In this way, the liquid fuel (191) can be added to the disc-like burner base (31) to flow through the first gap (312) into the second gap (314) and then to drip into the fuel trough (19) via the drip holes (317).

Referring to FIGS. 2, 3, 5 and 7, in one embodiment of the present invention, the second annular container (71) has an outer flange (712) extended around a top edge thereof. The second center tube (713) has a top end thereof terminating in a nozzle (7130), and also has an inner flange (7132) horizontally projected from an inside wall thereof. In some embodiments of the present invention, one side of the outer edge of the breathable film (75) can be bonded to the outer flange (712), and one side of the inner edge of the breathable film (75) can be bonded to the inner flange (7132).

Referring to FIGS. 2, 3, 5 and 7, in one embodiment of the present invention, the inner flange (515) is formed with a plurality of first protrusions (5150) on the surface, and the outer flange (517) is formed with a plurality of second protrusions (5170) on the surface.

The second annular container (71) is placed in the first annular groove (519) of the first annular container (51) of the thermal conducting device (5). The first protrusions (5150) are stopped at the bottom side of the outer flange (712). The second protrusions (5170) are stopped at the bottom side of the inner flange (7132). In this way, the fragrance capsule (7) is kept apart from the thermal conducting device (5) by a gap and will not be directly heated. The burner (3) heats the thermal conducting device (5). The thermal conducting device (5) does not directly heat the fragrance capsule (7), but the thermal radiant heat of the thermal conducting device (5) is transmitted to the fragrance capsule (7) to heat the fragrance substance (73) at a constant and stable temperature for effective and continuous release of fragrance. The fragrance substance (73) does not run out too quickly.\

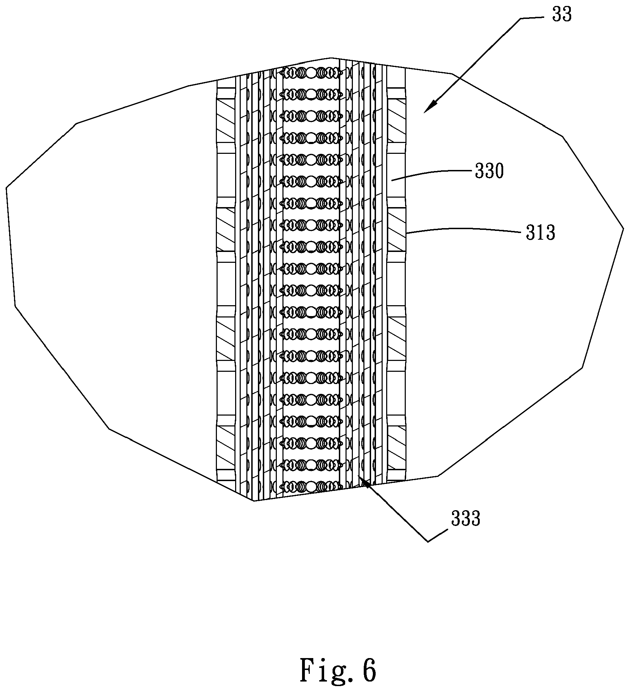

Referring to FIGS. 1-7, in one embodiment of the present invention, the first annular container (51) of the thermal conducting device (5) has a rim (512) provided at a top edge thereof. The aroma diffuser of the present invention further comprises an elastic cover (9) for covering the housing (1). The elastic cover (9) has a hook edge (91), a cover hole (93) and a plurality of dissipation holes (95). The hook edge (91) extends downwardly from the outer perimeter of the elastic cover (9). The cover hole (93) is formed at the center of the elastic cover (9). The dissipation holes (95) cut through opposing top and bottom sides of the elastic cover (9). The elastic cover (9) is detachably fastened to the first opening (511) of the thermal conducting device (5). The hook edge (91) is detachably and elastically hooked on the rim (512). The elastic cover (9) covers the first annular groove (519) and/or the fragrance capsule (7). The cover hole (93) is aimed at the first center tube (513) and the second center tube (713). The elastic cover (9), the thermal conducting device (5) and the fragrance capsule (7) are fastened together to form a donut structure. The elastic cover (9) with the thermal conducting device (5) and the fragrance capsule (7) can be taken integrally away from the housing (1). In this way, it is convenient to add the liquid fuel (191) to the disc-like burner base (31), and at the same time, the hook edge (91) of the elastic cover (9) can be easily removed from the thermal conducting device (5). The elastic cover (9) can be made of an elastic material such as silicone, rubber or plastic.

Referring to FIGS. 1-7, in one embodiment of the present invention, the breathable film (75) further comprises a layer of fixing film (751) and a layer of microporous film (755). The fixing film (751) is resistant to high temperatures and is not easily deformed. The fixing film (751) can fix the microporous film (755) against thermal deformation. The fixing film (751) has first pores. The microporous film (755) has second pores that are waterproof and moisture permeable. The fixing film (751) is made from polyester fiber, TETORON, or PET (polyethylene terephthalate). The microporous film (755) is made from TPE (thermoplastic elastomer), TPU (thermoplastic urethane), TPFE (polytetrafluoroethylene), polyethylene, polypropylene, or ultra-high molecular weight polyethylene.

The fixing film (751) is combined with the waterproof and moisture-permeable microporous film (755), and the fixing film (751) is used to fix the microporous film (755) without being deformed when heated, maintaining the breathable and waterproof function of the breathable film (75). The microporous film (755) is made from TPE (thermoplastic elastomer). The microporous film (755) that is made from TPE (thermoplastic elastomer) has second pores. The material of the fixing film (751) can be selected from polyester fiber. The fixing film (751) made from polyester fiber is combined with the microporous film (755) made from TPE (thermoplastic elastomer) to form the breathable film (75) that has a breathability. The aperture of the first pores is larger than the aperture of the second pores. The breathable film (75) is bonded to the second opening (711) of the second annular container (71). Using this layer of TPE microporous film (755) can achieve waterproof and moisture permeable effects, and also can maintain the quality of the fragrance inside of the fragrance capsule (7) during storage.

Referring to FIGS. 1-7, in one embodiment of the present invention, the burner (3) heating the thermal conducting device (5) and the fragrance capsule (7) can generate gas molecules and dissipate fragrance. The fixing film (751) does not undergo thermal expansion, contraction, deformation, etc. during the heating process. It can fix the TPE microporous film (755) against deformation. Therefore, the surface of the breathable film (75) will not deform and will not produce unsightly wrinkles. During the heating process, the fragrance substance (73) generates heat and has a concentration of aroma molecules, which flows through the second pores of the TPE microporous film (755) and the first pores of the fixing film (751) toward the low-concentration air outside the fragrance capsule (7), but the liquid (fluid) of the fragrance substance (73) will not pass through the second pores, and the use of the fragrance capsule (7) is safe.

Referring to FIGS. 1-7, in one embodiment of the present invention, the fragrance substance (73) includes, for example, any of a balm, a fragrance, a fragrance grease, a sesame oil, a sesame oil flavor mixture or an essential oil. In some embodiments of the present invention, the fragrance capsule (7) uses an aromatic wax, which has a hollow annular structure like a donut to fit in the second annular groove (719) of the second annular container (71).

Referring to FIGS. 1-7, in one embodiment of the present invention, the material of the second annular container (71) is, for example, a polymer. In some other embodiments of the present invention, the material of the second annular container (71) is plastic. The plastic is selected from, for example, any one of polyester plastic, PCTG (poly-cyclohexylenedimethylene terephthalate glycol), PET (polyethylene terephthalate), PTU (polythiourethane) or PP (polypropylene).

In all the above embodiments, the fixing film (751) of the fragrance capsule (7) and the microporous film (755) can be reversed upside down. The top side of the microporous film (755) is bonded with the fixing film (751) or the top side of the fixing film (751) is bonded with the microporous film (755).

In one embodiment of the present invention, the aperture of the first pores of the fixing film (751) is larger than or equal to the aperture of the second pores of the microporous film (755), facilitating passing of the aroma through the breathable film (75). That is, the air permeability per unit area of the first pores of the fixing film (751) is greater than or equal to the air permeability per unit area of the second pores of the microporous film (755), which further facilitates the passage of aroma through the breathable film (75).

In one embodiment of the present invention, the porosity of the first pores of the fixing film (751) is not equal to the porosity of the second pores of the microporous film (755).

The hollow part of the donut-shaped fragrance capsule (7) and the hollow part of the donut-shaped fragrance substance (73) will cause the fragrance substance (73) to dissolve and sublimate evenly and quickly when heated, while avoiding the central high temperature heat source. The thermal conducting device (5) heats the fragrance capsule (7) with a uniform temperature of radiant heat to prevent the fragrance substance (73) from heating up too quickly.

Although particular embodiments of the present invention have been described in detail for purposes of illustration, various modifications and enhancements may be made without departing from the spirit and scope of the invention. Accordingly, the invention is not to be limited except as by the appended claims.

## Claims

1. An aroma diffuser, comprising:
a housing (1) comprising an opening (11), an accommodation space (13), a plurality of air holes (15) and a locating portion (17), said opening (11) being formed in a top side of said housing (1), said accommodation space (13) being defined in said housing (1) in communication with said opening (11), said air holes (15) being formed on the periphery of said housing (1) below the elevation of said opening (11), said locating portion (17) being formed inside said housing (1) below said air holes (15);
a burner (3) comprising a disc-like burner base (31) and a wick (33), said disc-like burner base (31) comprising a wick hole (311) and a support tube (313), said support tube (313) being upwardly extended from said disc-like burner base (31) around said wick hole (311), said wick (33) being inserted into said support tube (313) and said wick hole (311), said burner (3) being detachably mounted on said locating portion (17) in said accommodation space (13) of said housing (1), said burner (3) defining with an inner bottom wall of said housing (1) a fuel trough (19) therebetween for the filling of a liquid fuel (191), said wick (33) having one end thereof inserted into said fuel trough (19) and an opposite end thereof extended out of said support tube (313) and suspending in said accommodation space (13) between said air holes (15); and
a thermal conducting device (5) detachably mounted on burner (3) in said accommodation space (13) of said housing (1).

2. The aroma diffuser as claimed in claim 1, further comprising a fragrance capsule (7) detachably mounted in said thermal conducting device (5), said fragrance capsule (7) holding therein a fragrance substance (73).

3. The aroma diffuser as claimed in claim 1, wherein said support tube (313) comprises a flange (316) extended around the periphery thereof above said wick hole (311); said disc-like burner base (31) further comprises a cover sheet (315) and a plurality of drip holes (317), said drip holes (317) being formed in a bottom side of said disc-like burner base (31), said cover sheet (315) having a center hole (319) attached onto said support tube (313), said cover sheet (315) being supported on said flange (316) of said support tube (313); a first gap (312) is left between the periphery of said cover sheet (315) and an inner bottom wall of said disc-like burner base (31), and a second gap (314) is left between said cover sheet (315) and said drip holes (317), so that the liquid fuel filled in said disc-like burner base (31) flows through said first gap (312), and then penetrates into said second gap (314) and drips into said fuel trough (19) via said drip holes (317).

4. The aroma diffuser as claimed in claim 3, wherein said wick (33) comprises a tube sleeve (331) and a metal mesh rod (333), said tube sleeve (331) having a plurality of holes (330) formed in a sleeve wall thereof, said metal mesh rod (333) being mounted in said tube sleeve (331) to absorb said liquid fuel (191) from fuel trough (19) by capillary adsorption.

5. The aroma diffuser as claimed in claim 4, wherein said metal mesh rod (333) is selectively a wick structure with a metal mesh wound around a central axis, or a wick structure formed by folding or stacking a metal mesh into shape.

6. The aroma diffuser as claimed in claim 3, wherein said thermal conducting device (5) comprises a first annular container (51), said first annular container (51) comprising a first opening (511), a first center tube (513) and an inner flange (515), said first opening (511) being formed in a top side of said first annular container (51), said inner flange (515) horizontally projecting from an inside wall of said first annular container (51), said first center tube (513) upwardly projecting from an inner bottom wall of said first annular container (51) and being provided with an outer flange (517) around the periphery thereof, said first center tube (513) and said first annular container (51) defining therebetween a first annular groove (519).

7. The aroma diffuser as claimed in claim 6, further comprising a fragrance capsule (7), said fragrance capsule (7) comprising a second annular container (71) placed in said first annular groove (519) of said first annular container (51) of said thermal conducting device (5), a fragrance substance (73) and a breathable film (75), said second annular container (71) comprising a second opening (711) and a second center tube (713), said second opening (711) being formed in a top side of said second annular container (71), said second center tube (713) having a top end thereof terminating in a nozzle (7130), said second center tube (713) upwardly projecting from an inner bottom wall of said second annular container (71), said second center tube (713) and said second annular container (71) defining therebetween a second annular groove (719); said fragrance substance (73) is mounted in said second annular groove (719); said breathable film (75) is breathable and waterproof and bonded to said second opening (711) of said second annular container (71) to cover said fragrance substance (73).

8. The aroma diffuser as claimed in claim 7, wherein said second annular container (71) comprises an outer flange (712) extended around a top edge thereof; said second center tube (713) has a top end thereof terminating in a nozzle (7130), and also has an inner flange (7132) horizontally projected from an inside wall thereof, wherein said inner flange (515) comprises a plurality of first protrusions (5150) protruded from a surface thereof; said outer flange (517) comprises a plurality of second protrusions (5170) protruded from a surface thereof; said second annular container (71) is placed in said first annular groove (519) of said first annular container (51) of said thermal conducting device (5); said first protrusions (5150) are stopped against said outer flange (712) said second annular container (71), and said second protrusions (5170) are stopped against said inner flange (7132) of said second center tube (713), so that a gap is left between said fragrance capsule (7) and said thermal conducting device (5).

9. The aroma diffuser as claimed in claim 7, wherein said breathable film (75) comprises a fixing film (751) and a microporous film (755), said fixing film (751) having a plurality of first pores, said microporous film (755) having a plurality of second pores, said fixing film (751) and said microporous film (755) being bonded together to form said breathable film (75).

10. The aroma diffuser as claimed in claim 9, wherein said fixing film (751) is selectively made from polyester fiber, TETORON, or PET (polyethylene terephthalate); said microporous film (755) is selectively made from TPE (thermoplastic elastomer), TPU (thermoplastic urethan), TPFE (polytetrafluoroethylene), polyethylene, polypropylene, or ultra-high molecular weight polyethylene.

11. The aroma diffuser as claimed in claim 7, wherein said first annular container (51) of said thermal conducting device (5) comprises a rim (512) extended around a top edge thereof; the aroma diffuser further comprises an elastic cover (9) for covering said housing (1), said elastic cover (9) comprising a hook edge (91), a cover hole (93) and a plurality of dissipation holes (95), said hook edge (91) extending downwardly from the outer perimeter of said elastic cover (9), said cover hole (93) being formed at the center of said elastic cover (9), said dissipation holes (95) cutting through opposing top and bottom sides of said elastic cover (9), said elastic cover (9) being detachably fastened to said first opening (511) of said thermal conducting device (5), said hook edge (91) being detachably and elastically hooked on said rim (512), said elastic cover (9) covering said first annular groove (519) and/or said fragrance capsule (7).

12. A fragrance capsule (7), comprising a second annular container (71), a fragrance substance (73) and a breathable film (75), said second annular container (71) comprising a second opening (711) and a second center tube (713), said second opening (711) being formed in a top side of said second annular container (71), said second center tube (713) having a top end thereof terminating in a nozzle (7130), said second center tube (713) upwardly projecting from an inner bottom wall of said second annular container (71), said second center tube (713) and said second annular container (71) defining therebetween a second annular groove (719); said fragrance substance (73) is placed in said second annular groove (719); said breathable film (75) is breathable and waterproof and bonded to said second opening (711) of said second annular container (71) to cover said fragrance substance (73); said second annular container (71) comprises an outer flange (712) extended around a top edge thereof; said second center tube (713) comprises an inner flange (7132) horizontally projected from an inside wall thereof.

13. The fragrance capsule (7) as claimed in claim 12, wherein said breathable film (75) comprises a fixing film (751) and a microporous film (755), said fixing film (751) having a plurality of first pores (753), said microporous film (755) having a plurality of second pores (757), said fixing film (751) and said microporous film (755) being bonded together to form said breathable film (75), the aperture of said first pores being larger than the aperture of said second pores.

14. The fragrance capsule (7) as claimed in claim 13, wherein said fixing film (751) is selectively made from polyester fiber, TETORON, or PET (polyethylene terephthalate); said microporous film (755) is selectively made from TPE (thermoplastic elastomer), TPU (thermoplastic urethane), TPFE (polytetrafluoroethylene), polyethylene, polypropylene, or ultra-high molecular weight polyethylene.

15. The fragrance capsule (7) as claimed in claim 13, wherein said fragrance substance (73) is a fragrance wax having a hollow annular structure.
